# EUROPEAN PATENT APPLICATION

(11) **EP 3 822 344 A1**
(43) Date of publication of application: **19.05.2021**
(21) Application number: 18919224.8
(22) Date of filing: 04.06.2018
(51) Int. Cl.: C12N 5/10, A61K 35/14, A61P 37/02

(54) **METHOD FOR PREPARING CHIMERIC ANTIGEN RECEPTOR T CELLS BY SERUM-FREE CULTURE**

(30) Priority: 16.05.2018 CN 201810467017
(71) Applicant: Cellular Biomedicine Group HK Limited, Admiralty, Hong Kong (HK)
(72) Inventor: ZHAO, Dijun, Shanghai 201210 (CN); LI, Chao, Shanghai 201210 (CN); WANG, Fei, Shanghai 201210 (CN); WU, Junfeng, Shanghai 201210 (CN); LIU, Xiaoyu, Shanghai 201210 (CN); ZHAO, Jiawei, Shanghai 201210 (CN)
(74) Representative: Cabinet Becker et Associés
(86) International application number: PCT/CN2018/089801
(87) International publication number: WO 2019/218402

(57) **Abstract**

Provided is a method of preparing chimeric antigen receptor T cells by serum-free culture. The method comprises steps of: (a) providing PBMC cells; (b) performing negative sorting treatment on the PBMC cells to obtain sorted PBMC cells; (c) activating the sorted PBMC cells to obtain activated T cells; (d) transfecting the activated T cells with a viral vector expressing a chimeric antigen receptor, so as to obtain transfected T cells; (e) removing the viruses from the transfected T cells to obtain virus-removed T cells; and (f) subjecting the virus-removed T cells to expansion culture to obtain chimeric antigen receptor T cells.

## Description

### Technical Field

The present invention relates to the field of biological technology, particularly to a method of preparing chimeric antigen receptor T cells by serum-free culture.

### Background Art

Chimeric antigen receptor T cell immunotherapy is a new type of medical treatment technology that causes T cells to have scFv fragments and intracellular signal domains, which can specifically recognize tumor-associated antigens by means of gene editing, thereby enhancing T cell targeting, killing and durability. In recent years, the chimeric antigen receptor T cell immunotherapy has had good performance in clinical tumor immunotherapy, bringing hope for clinical cure of tumors.

The preparation process of chimeric antigen receptor T cells is relatively complex and involves many operations such as cell activation, sorting, transfection and expansion culture. Insufficiency of any link such as process flow, facilities, and reagent selection will have an important impact on the quality of cell preparation, which in turn will affect the vitality, yield, safety and subsequent clinical effects of chimeric antigen receptor T cells.

Serum, as an important component in a conventional cell culture process, contains some substances that have toxic and side effects on cells. Further, the quality of serum varies greatly with batches. In addition, mycoplasma, viruses and other risks may be brought into the process of serum collection, which seriously hinder the development of cell therapy.

At present, there is no satisfactory and efficient process for serum-free preparation of chimeric antigen receptor T cells on the market, seriously affecting the further development, promotion and application of chimeric antigen receptor T cell immunotherapy.

Therefore, there is an urgent need in the art to develop a safe and efficient method of preparing chimeric antigen receptor T cells based on serum-free cell culture.

### SUMMARY OF THE INVENTION

An object of the present invention is to provide a safe and efficient method of preparing chimeric antigen receptor T cells based on serum-free cell culture.

In a first aspect of the present invention, a serum-free method of preparing chimeric antigen receptor T cells is provided:
(a) providing PBMC cells, which are resuspended in a serum-free medium;
(b) performing negative sorting treatment on the PBMC cells at step (a) to obtain sorted PBMC cells;
(c) activating the sorted PBMC cells obtained at step (b) to obtain activated T cells;
(d) performing gene transfection on the activated T cells obtained at step (c) with a viral vector expressing a chimeric antigen receptor to obtain transfected T cells;
(e) removing the viruses from the transfected T cells to obtain virus-removed T cells; and
(f) resuspending the virus-removed T cells in an expansion medium and performing expansion culture to obtain chimeric antigen receptor T cells, and harvesting the chimeric antigen receptor T cells when the chimeric antigen receptor T cells reach a predetermined quantity, wherein the expansion medium is a serum-free medium containing a cell factor.

In an alternative preferred embodiment, a serum-free medium is used at all steps of the method.

In an alternative preferred embodiment, at step (a), the PBMC cells are resuscitated PBMC cells.

In an alternative preferred embodiment, the PBMC cells are autologous or allogeneic.

In an alternative preferred embodiment, the PBMC cells are derived from human.

In an alternative preferred embodiment, at step (b), the negative sorting treatment uses CliniMACS.

In an alternative preferred embodiment, at step (c), the anti-CD3/CD28 antibody for activation performs activation treatment.

In an alternative preferred embodiment, at step (c), the activation treatment is performed in an activation medium, which contains a basic medium and an additive.

In an alternative preferred embodiment, the basic medium is selected from the following group: LONZA X-VIVO,LIFE CTS AIM V, LIFE OpTmizer SFM, RPMI 1640, ImmunoCult™ -XF and Stemlineä.

In an alternative preferred embodiment, the additive is selected from the following group: human serum albumin, recombinant human serum albumin, plant-derived recombinant albumin or a combination thereof, preferably recombinant albumin.

In an alternative preferred embodiment, the albumin concentration is 0.01% to 50% (W/V), preferably 0.05% to 30% (W/V), more preferably, 0.1% to 20% (W/V).

In an alternative preferred embodiment, the activation treatment is selected from the following group: antibody coating treatment, free antibody treatment, activating magnetic bead treatment or a combination thereof.

In an alternative preferred embodiment, in the activation treatment, the antibody concentration is 10 ng/ml to 100 ng/ml.

In an alternative preferred embodiment, in the activation treatment, the ratio of the number of activating magnetic beads to cells is 0.25∼5: 1, preferably; 0.5∼3: 1.

In an alternative preferred embodiment, the time of the activation treatment is 3 to 8 days.

In an alternative preferred embodiment, at step (d), the viral vector is selected from the following group: lentivirus, adeno-associated virus (AAV), adenovirus or a combination thereof.

In an alternative preferred embodiment, the multiplicity of infection (MOI) of the gene transfection is 0.5 to 30, preferably 1 to 20, more preferably 2 to 10.

In an alternative preferred embodiment, at step (e), the virus removal treatment includes: resuspending the transfected cells in a serum-free medium, centrifuging the cells and sucking up the supernatant to obtain virus-removed T cells.

In an alternative preferred embodiment, at step (f), the predetermined quantity is 1×10⁹ to 1×10¹¹, preferably 2×10⁹ to 2×10¹⁰.

In an alternative preferred embodiment, the expansion medium at step (f) further contains a cell factor.

In an alternative preferred embodiment, the cell factor is selected from the following group: IL-2, IL-15, IL-7 or a combination thereof.

In an alternative preferred embodiment, the concentration of each cell factor is independently I to 100 ng/ml, preferably 2 to 80 ng/ml, more preferably, 5 to 50 ng/ml.

In an alternative preferred embodiment, at step (f), the expansion culture is performed in a container, which is selected from the following group: a culture flask, a culture bag or a combination thereof.

In an alternative preferred embodiment, the culture flask is selected from the following group: G-Rex culture flask, T75 culture flask or a combination thereof.

In an alternative preferred embodiment, the container is a G-Rex culture flask.

In an alternative preferred embodiment, the time of the expansion culture is 8 to 14 days.

In an alternative preferred embodiment, at step (f), in the expansion culture, no operation of changing the container is performed.

In an alternative preferred embodiment, the chimeric antigen receptor directs at a target selected from the following group: CD19, CD23, etc.

In a second aspect of the present invention, chimeric antigen receptor T cells are provided. The chimeric antigen receptor T cells are prepared by the serum-free method of preparing chimeric antigen receptor T cells as described in claim 1.

In a third aspect of the present invention, a cell preparation is provided, which contains (a) the chimeric antigen receptor T cells in claim 2 and (b) a pharmaceutically acceptable carrier.

In an alternative preferred embodiment, the cell preparation is a liquid preparation (e.g., an injection).

It should be understood that within the scope of the present invention, the foregoing technical features of the present invention and the technical features specifically described below (e.g., embodiments) can be combined with each other to form a new or preferred technical solution.

Due to space limitation, they are not described one by one.

### BRIEF DESCRIPTION OF THE DRAWINGS

Fig. 1 shows changes in a growth curve of CAR-T cells in different media.
Fig. 2 shows changes in a growth curve of CAR-T cells in a G-Rex culture flask, a culture bag and a T75 culture flask.
Fig. 3 shows changes in a positive rate curve of CAR-T cells in a G-Rex culture flask, a culture bag and a T75 culture flask.
Fig. 4 shows effects of different cell culture additives on the proliferation of CAR-T cells.
Fig. 5 shows the proliferation rates of cells in different sorting methods.

### DETAILED DESCRIPTION

After extensive and in-depth research, the inventors developed a unique and innovative process of serum-free culture for preparing chimeric antigen receptor T cells for the first time. Based on the serum-free culture method of the present invention, not only the success rate and yield of culture of chimeric antigen receptor T cells can be raised but also the toxic side effects of serum on CAR-T cells can be avoided and the risks introduced due to serum can be reduced significantly or eliminated, so as to provide help for the further development and promotion of chimeric antigen receptor T cell immunotherapy. On this basis, the present invention is completed.

### Terminology

### Method

In the present invention, positive sorting or negative sorting can be used. A preferred sorting method is a sorting method based on MACS. For example, CliniMACS technology can be used for sorting.

MACS is a highly specific cell sorting technology. The main components include MACS microspheres, an MACS sorting column and an MACS separator. The MACS microspheres are superparamagnetic particles coupled with highly specific monoclonal antibodies. The MACS sorting column is placed in an MACS separator of a permanent magnetic field. The negative sorting is a method of removing magnetic markers in non-target cells from a cell mixture, that is, non-magnetically labeled cells are target cells.

Preferably, the method of the present invention adopts CliniMACS or CliniMACSplus sorting technology (and device) for negative sorting, so as to obtain the desired target cells.

Typically, the sorted cells in the present invention are essentially composed of CD3 positive cells.

### Chimeric antigen receptor (CAR)

As used herein, a chimeric antigen receptor (CAR) comprises an extracellular domain, an optional hinge region, a transmembrane domain and an intracellular domain. The extracellular domain comprises optional signal peptides and target-specific binding elements (also known as antigen binding domains). The intracellular domain comprises costimulatory molecules and ζ chain parts. When CAR is expressed in T cells, the extracellular segment can recognize a specific antigen and then transduce the signal through the intracellular domain, causing cell activation and proliferation, cytolytic toxicity, and secretion of cell factors such as IL-2 and IFN-γ, affecting tumor cells, causing the tumor cells to not grow, or to be promoted to die or affected in other ways, and leading to a reduced or eliminated tumor burden on the patient. The antigen binding domain is preferably fused with one or more intracellular domains from the costimulatory molecule and the ζ chain.

### Preparation

The present invention provides a cell preparation, specifically as described in the third aspect of the present invention. In an implementation manner, the cell preparation contains (a) the chimeric antigen receptor T cells described in the second aspect of the present invention and (b) a pharmaceutically acceptable carrier. In an implementation manner, the cell preparation is a liquid preparation (e.g., an injection).

### The present invention has the following main advantages:

(a) Compared with conventional cell culture, the culture method of the present invention adopts culture systems such as serum-free medium and G-Rex to avoid introducing substances that have toxic side effects on cells and to greatly improve the success rate and safety of the culture of chimeric antigen receptor T cells.
(b) The culture method of the present invention adopts a specially optimized process flow, thereby significantly improving the effectiveness of the culture of chimeric antigen receptor T cells, particularly the positive rate and yield of harvested CAR-T cells.

The present invention will be further described in conjunction with specific embodiments. It should be understood that these embodiments are intended to illustrate the present invention only and not to limit the scope of the present invention. The experimental methods, without indicating specific conditions in the following embodiments, normally follow conventional conditions, such as the conditions described in Sambrook et al., Molecular Cloning: Laboratory Manual (New York: Cold Spring Harbor Laboratory Press, 1989), or according to the conditions recommended by the manufacturer. Unless otherwise stated, percentages and parts are percentages by weight and parts by weight.

### Reagent

Serum-free medium: basic medium + albumin

### Embodiment 1

### Medium selection

### 1.1 Cryopreserved or fresh PBMC

Take cryopreserved or fresh PBMC with a cell count of 300×10⁶ to 1000×10⁶ and resuspend the PBMC in a serum-free medium.

### 1.2 Sorting

Use CD19 and CD14 labeling magnetic beads to label and incubate the PBMC for 30 minutes, install the pipes needed by sorting on CliniMACS according to the requirements, and after completion of the cell incubation, perform the operation of negative sorting to remove CD19- and CD14-labeled impurity cells and obtain sorted cells with CD3 positive cells as the main component.

### 1.3 Cell activation

Use CD3/CD28 to activate the cells obtained from sorting and then use a serum-free medium (supplemented with IL-2) containing albumin at a final concentration of 1.0% to perform inoculated culture at a cell density of 3×10⁶ to 6×10⁶/ml for 2 days.

### 1.4 Gene transduction

Centrifuge the cells, discard the supernatant, then add the required volume of lentivirus according to MOI 2-10, resuspend in a serum-free medium (containing albumin at a final concentration of 1.0%) and then transfer the liquid to a culture flask and culture it at 37°C, 5% CO₂ (12 to 48 hours).

Here, the lentivirus is a viral vector expressing the target CAR gene.

### 1. 5 Virus removal

Centrifuge the cell suspension at 200 to 300 g for 6 to 8 minutes. Suck and discard the supernatant and resuspend the cells, then transfer the cells to a serum-free medium (containing albumin at a final concentration of 1.0%) and add IL-2 to a concentration of 50 to 500 IU/ml, thereby obtaining virus-removed T cells.

### 1.6 Expansion culture

Transfer the virus-removed T cells to a G-Rex culture flask and perform expansion culture at 37°C, 5% CO₂.

After 3 to 10 days of expansion culture, take the G-Rex bottle out of the incubator, mix the cells well, take a sample and count the cells, supplement IL-2 to a concentration of 50 to 500 IU/ml, continue the culture and harvest when the cell yield reaches 1×10⁹ to 1×10¹⁰ CAR-T cells.

### 1.7 Results

It was determined that in the harvested cells, the CAR-T positive rate was greater than 20%.

### Embodiment 2.

### Selection of experimental consumables

### 2.1 PBMC resuscitation, sorting, cell activation and gene transduction

Use the same method as in Embodiment 1 for resuscitation, sorting, cell activation, gene transduction of cryopreserved PBMC.

### 2.2 Virus removal

Centrifuge the foregoing cell suspension at 200 to 300 g for 6 to 8 minutes. Suck and discard the supernatant, and flick to resuspend the cells, so as to obtain virus-removed T cells.

### 2.3 Expansion culture

Transfer the virus-removed T cells to a medium (OpTmizer SFM+1.0% albumin), add IL-2 (the final concentration is 25 IU/ml), then transfer to a G-Rex culture flask, a culture bag and a T75 culture flask respectively and then continue the culture at 37°C, 5% CO₂.

After continuing the culture for three days, take samples from the G-Rex culture flask, the culture bag and the T75 culture flask respectively, count the cells and record cell density and cell viability. Reserve samples for testing.

Supplement IL-2 (the final concentration is 25 IU/ml) and then put back into the incubator for continued culture.

After continuing the culture for one day, take samples from the G-Rex culture flask, the culture bag and the T75 culture flask respectively, count the cells and record cell density and cell viability. Reserve samples for testing.

After continuing the culture for two days, take samples from the G-Rex culture flask, the culture bag and the T75 culture flask respectively, count the cells and record cell density and cell viability. Reserve samples for testing.

### 2.4 Results

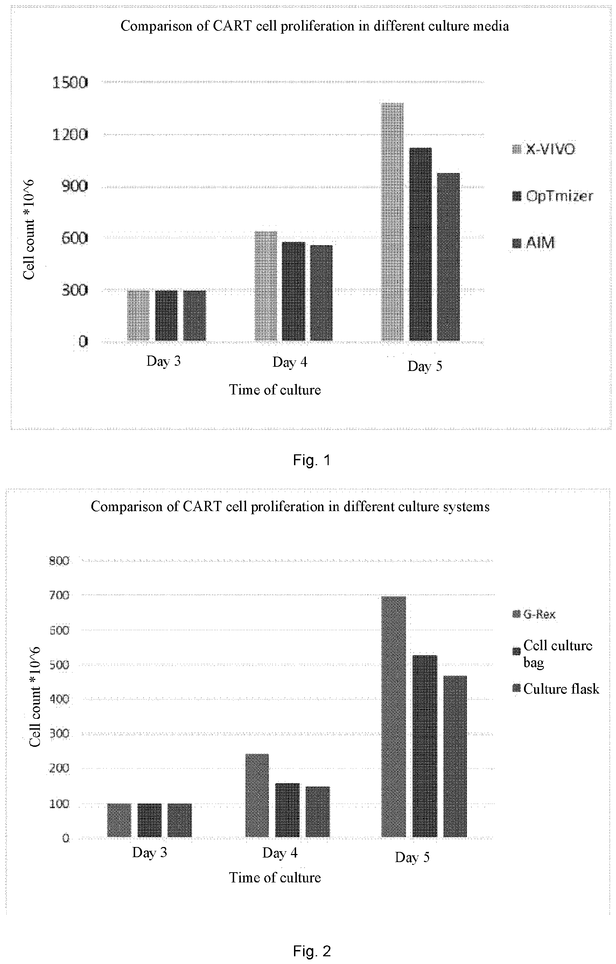

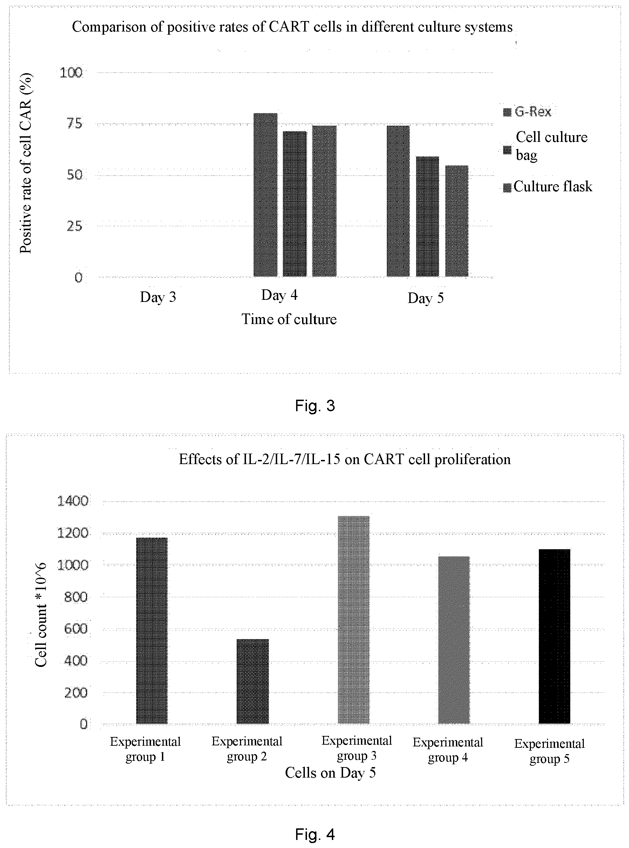

As shown in Fig. 2, the CAR-T cells showed a faster cell proliferation rate from Day 3 to Day 5 in the G-Rex culture system than in the culture bag and the culture flask; as shown in Fig. 3, the CAR-T cells were able to maintain a high level of CAR positive rate in G-Rex and the decent degree was smaller than those in the cell culture bag and the culture flask; in summary, if a large number of CAR-T positive cells are needed in a short time, G-Rex has the advantage of a higher proliferation rate and a higher positive rate over the cell culture bag and culture flask.

### Embodiment 3.

### Cell culture additive experiment

### 3.1 Method

In this embodiment, different additives IL-2, IL-7, IL-15 or a combination thereof were added to a cell culture medium, and there were five experimental groups in total.

**Table 1**

| | Experimental group 1 | Experimental group 2 | Experimental group 3 | Experimental group 4 | Experimental group 5 |
|---|---|---|---|---|---|
| IL-2 | + | - | + | + | + |
| IL-7 | - | + | + | + | - |
| IL-15 | - | + | + | - | + |

Use the same method as in Embodiment 1 to perform resuscitation, sorting, magnetic bead-labeled activation, gene transduction, magnetic bead removal, virus removal and expansion culture of cryopreserved PBMC.
1) The medium used was OpTmizer SFM+albumin (the final concentration was 1.0%).
2) All the steps of adding IL-2 were replaced with the cell factors corresponding to experimental groups 2 to 5 in Table 1 for culture.

### 3.2 Results

As shown in Fig. 4, combination of cell factors IL-2, IL-7 and IL-15 in a culture system had the best effect on increase of cell proliferation and helped to increase the quantity of CAR-T cells.

### Embodiment 4.

### Comparative experiment of positive sorting and negative sorting

### 4.1 Study object:

Comparison of the effects of the same PBMC on cell culture using a positive sorting method and a negative sorting method respectively.

### 4.2 Cell sorting:

Resuspend the resuscitated PBMC in a sorting buffer and divide it into two equal parts and perform negative sorting CD19+CD14+ and positive sorting CD3+ respectively.

### 4.3 Cell activation:

Inoculate the sorted cells with a culture medium, add activating magnetic beads, mix them well and culture them.

### 4.4 Virus transfection:

Calculate the number of activated cells when the culture reaches the second day, calculate the required number of lentiviral vectors based on the MOI (2∼10), take the corresponding lentiviral vector, evenly resuspend it in a culture medium, centrifuge to remove the original cell supernatant and add the lentiviral vector suspension for resuspension and continue the culture.

### 4.5 Remove virus and activating magnetic beads:

On the third day of the culture, remove the virus by centrifugation and remove the activating magnetic beads by a magnetic grate and add a culture medium to continue the culture.

Conduct testing on the eighth day of the culture.

### 4.6 Results

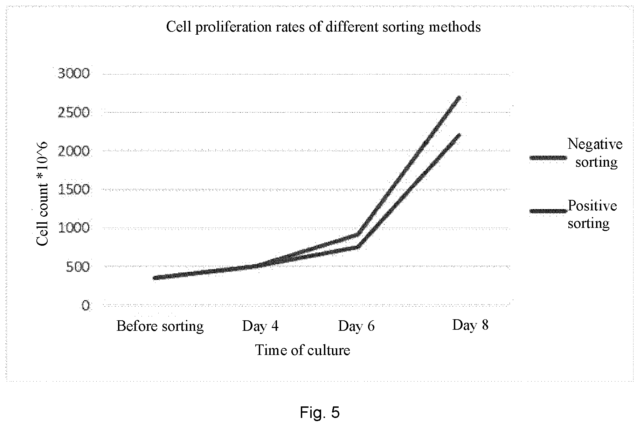

The results are as shown in Table 2 and Fig. 5:

**Table 2**

| | After sorting | | | | | The 8^{th} day of culture | | | | |
|---|---|---|---|---|---|---|---|---|---|---|
| | CD3 + | CD19+ | CD14+ | Red blood cell | Others | CD3 + | CD19+ | CD14+ | Red blood cell | Others |
| Negative sorting | 60% | 2% | 1% | 34% | 3% | 98% | 1% | 0% | 0% | 1% |
| Positive sorting | 100% | 0% | 0% | 0% | 0% | 100% | 0% | 0% | 0% | 0% |

The ratio of target cells collected from PBMC by the method of negative sorting was close to the ratio of target cells collected from PBMC by the method of positive sorting, but as for the process of cell proliferation, the method of negative sorting showed an obvious increase compared with the method of positive sorting. In summary, the use of the method of negative sorting eventually led to harvesting of more positive cells CD3.

All the documents mentioned in the present invention have been cited herein as references, as if each document is individually cited as a reference. Further, it should be understood that after reading the above-taught content of the present invention, those skilled in the art may make various changes or modifications to the present invention and these equivalent forms will also fall within the scope delimited by the claims of the present application.

## Claims

1. A serum-free method of preparing chimeric antigen receptor T cells, wherein the method comprises steps of:
(a) providing PBMC cells, which are resuspended in a serum-free medium;
(b) performing negative sorting treatment on the PBMC cells at step (a) to obtain sorted PBMC cells;
(c) activating the sorted PBMC cells obtained at step (b) to obtain activated T cells;
(d) performing gene transfection on the activated T cells obtained at step (c) with a viral vector expressing a chimeric antigen receptor to obtain transfected T cells;
(e) removing the viruses from the transfected T cells to obtain virus-removed T cells; and
(f) resuspending the virus-removed T cells in an expansion medium and performing expansion culture to obtain chimeric antigen receptor T cells, and harvesting the chimeric antigen receptor T cells when the chimeric antigen receptor T cells reach a predetermined quantity, wherein the expansion medium is a serum-free medium containing a cell factor.

2. The method according to claim 1, wherein a serum-free medium is used at all steps.

3. The method according to claim 1, wherein at step (c), the activation treatment is performed in an activation medium, which contains a basic medium and an additive.

4. The method according to claim 1, wherein the basic medium is selected from the following group: LONZA X-VIVO, LIFE CTS AIM V, LIFE OpTmizer SFM, RPMI 1640, ImmunoCult™-XF and Stemlinea.

5. The method according to claim 1, wherein the additive is selected from the following group: human serum albumin, recombinant human serum albumin, plant-derived recombinant albumin or a combination thereof, preferably recombinant albumin.

6. The method according to claim 1, wherein the expansion medium at step (f) further contains a cell factor

7. The method according to claim 1, wherein the cell factor is selected from the following group: IL-2, IL-15, IL-7 or a combination thereof.

8. The method according to claim 1, wherein at step (f), the expansion culture is performed in a container, which is selected from the following group: a culture flask, a culture bag, or a combination thereof.

9. Chimeric antigen receptor T cells, wherein the chimeric antigen receptor T cells are prepared by the serum-free method of preparing chimeric antigen receptor T cells as described in claim 1.

10. A cell preparation, wherein the cell preparation contains (a) the chimeric antigen receptor T cells in claim 2 and (b) a pharmaceutically acceptable carrier.
